# EUROPEAN PATENT APPLICATION

(11) **EP 1 785 147 A2**
(43) Date of publication of application: **16.05.2007**
(21) Application number: 06023482.0
(22) Date of filing: 10.11.2006
(51) Int. Cl.: A61L 2/18, A61L 2/26, A61B 1/12

(54) **Apparatus for washing and disinfecting endoscope**

(30) Priority: 11.11.2005 JP 2005327890
(71) Applicant: OLYMPUS MEDICAL SYSTEMS CORP., Shibuya-ku Tokyo 151-0072 (JP)
(72) Inventor: Noguchi, Toshiaki, Tachikawa-shi Tokyo 190-0002 (JP); Suzuki, Eiri, Sagamihara-shi Kanagawa-ken 229-0038 (JP); Kuroshima, Hisashi, Hachiouji-shi Tokyo 193-0832 (JP); Onisihi, Hideo, Hachiouji-shi Tokyo 192-0045 (JP)
(74) Representative: von Hellfeld, Axel

(57) **Abstract**

There is provided an apparatus for washing and disinfecting an endoscope (20) equipped with an insertion tube through which a duct (22a) is formed and a base portion (21) integrated with the insertion tube. The apparatus comprises a bath member (3) providing a washing bath (5); and an accommodating member (10) on which the endoscope is accommodated. The accommodating member is installed in the washing bath. The apparatus further comprises a duct washing unit (16c, 17c, 27, 26, 30, 41-60, 57A, 62-70) and a brush washing unit (26, 41-59, 78, 79, 84, 85). The duct washing unit is equipped with a washing brush (27a) and formed to wash the duct by advancing and retreating the brush through the duct, with the endoscope accommodated on the accommodating member. The brush washing unit is configured to wash the brush by spraying fluid toward the brush when the brush is made to advance and retreat through the duct.

## Description

### CROSS REFERENCE TO RELATED APPLICATION

The patent application related to and incorporates by reference Japanese Patent application No. 2005-327890 filed on Nov. 11, 2005.

### Background of the Invention

### (The field of the Invention)

The present invention relates to an apparatus for washing and disinfecting used endoscopes, and in particular, to a washing and disinfecting apparatus with a brush for washing ducts formed in an endoscope.

### (Related art)

An endoscope used for inspecting and treating an object being examined has a tubular portion (called "insertion tube") inserted into body cavities of an object being examined. The insertion tube is provided with various inner ducts formed inside therethrough, in which such ducts (hereinafter referred to as endoscopic channels) include a channel for suction which serves as a forceps channel as well. Once being used, the insertion tube is polluted by humor, such as mucosa and blood, and filth attached to the outer surface as well as the endoscope channel (ducts). Thus, it is necessary to sufficiently wash and disinfect the insertion tube, that is, the outer surface and the endoscopic channel of the insertion tube, every time the endoscope is used.

Japanese Patent Laid-open Publication No. 2003-10118 proposes one technique to the foregoing demand by disclosing an endoscope washing apparatus with a brush for washing endoscopic channels. This endoscope washing apparatus has means for washing an endoscopic channel for forceps, in which the washing means is composed of a washing wire brush which automatically goes back and forth along the channel and a brush driver for driving the wire brush to enable such reciprocating motions.

Another technique is provided by Japanese Patent Laid-open Publication No. 2004-16617, which proposes an improved technique which uses a washing brush similar to that disclosed in the foregoing known publication (Japanese Patent Laid-open Publication No. 2003-10118). In this improved technique, to avoid humor and filth, which are dropped from the wall, from being left in the endoscopic channels, a washing fluid is fed through the endoscopic channels at a speed faster than the reciprocating speed of a washing brush.

However, the washing technique is still confronted with a difficulty in that it is difficult to completely get rid of dirty matters, such as humor and filth, stuck to the brush itself. Thus, to realize a more steadier removal of such pollutants from the brush, the washing brush should be subjected again to a manual kneading and washing process after the washing of the endoscope.

Accordingly, in the conventional endoscope washing apparatuses, the washing process of endoscopes always involves the manual kneading and washing process of brushes used for brushing. Such manual treatment needs a long work time and imposes a troublesome work on workers. In addition, how the brushes are washed and how long the washing work is done depends on the workers who are engaged in such jobs. There is still a concern about the sufficient removal of the pollutants from the brushes.

The kneading and washing process of the brushes gives rise to a loss in time in washing a succeeding endoscope, affecting the operating rate of each endoscope.

### Summary of the Invention

The present invention has been made in consideration of the foregoing conventional situations, and has an object to provide an endoscope washing and disinfecting apparatus which is capable of washing and disinfecting used endoscopes including their endoscopic channels (ducts) in a sanitary and effective manner with no manual work imposed on the workers.

The present invention provides an apparatus for washing and disinfecting an endoscope (20) equipped with an insertion tube through which a duct (22a) is formed and a base portion (21) integrated with the insertion tube. The apparatus comprises a bath member (3) providing a washing bath (5); and an accommodating member (10) on which the endoscope is accommodated. The accommodating member is installed in the washing bath. The apparatus further comprises a duct washing unit (16c, 17c, 27, 26, 30, 41-60, 57A, 62-70) and a brush washing unit (26, 41-59, 78, 79, 84, 85). The duct washing unit is equipped with a washing brush (27a) and formed to wash the duct by advancing and retreating the brush through the duct, with the endoscope accommodated on the accommodating member. The brush washing unit is configured to wash the brush by spraying fluid toward the brush when the brush is made to advance and retreat through the duct.

According to the present invention, when an operator accommodates an endoscope on the accommodating member, the brush is subjected to the spray of the fluid performed by the brush washing unit. That is, the brush, which is for washing the duct of the endoscope, is mechanically washed as well. Thus, the duct of a used endoscope can be washed and disinfected in a steady, sanitary, and labor-efficient manner. The operator can be released from troublesome manual work for washing the brush.

### Brief Description of the Drawings

In the accompanying drawings:
Fig. 1 is a perspective view outlining the overall configuration of an endoscope washing and disinfecting apparatus (with a top cover open) according to an example of the present invention;
Fig. 2 is a perspective view showing the endoscope washing and disinfecting apparatus, in which an endoscope is accommodated and the top cover is closed;
Fig. 3 is a schematic view showing an outlined duct structure of the apparatus;
Fig. 4 is a plan view showing a nozzle-mounted device of the apparatus;
Fig. 5 is a plan view showing a tray on which an endoscope is accommodated ;
Fig. 6 is a plan view showing a washing bath;
Fig. 7 is a partial sectional view showing a connection between a washing nozzle mounted to the tray and a nozzle connector mounted to a base of the washing bath;
Fig. 8 illustrates a condition where a brush advances so as to protrude from a suction channel and positioned just above the nozzle;
Fig. 9 is a partial sectional view showing a connection between a washing nozzle mounted to the base of the washing bath, which is according to a modification;
Fig. 10 illustrates a condition where a brush advances so as to protrude from a suction channel and positioned just above the nozzle, which is according to the modification shown in Fig. 9;
Fig. 11 is a schematic diagram showing a control system for selectively driving a nozzle among a plurality of nozzles;
Fig. 12 is a plan view showing a state where an endoscope having the longest insertion tube is accommodated on the tray;
Figs. 13 to 15 are plan view each showing a state where another endoscope having a different-length insertion tube is accommodated;
Fig. 16 shows another modification concerning another location of the nozzle;
Fig. 17 is a side view explaining the positional relationship between the brush and the nozzle in the location shown in Fig. 16; and
Fig. 18 exemplifies the processing carried out by a controller to selectively drive a nozzle depending on the length of the insertion tube of an endoscope being washed.

### Detailed Description of the Preferred Embodiments

Various embodiments of the present invention will now be described with reference to the accompanying drawings.

### (First Embodiment)

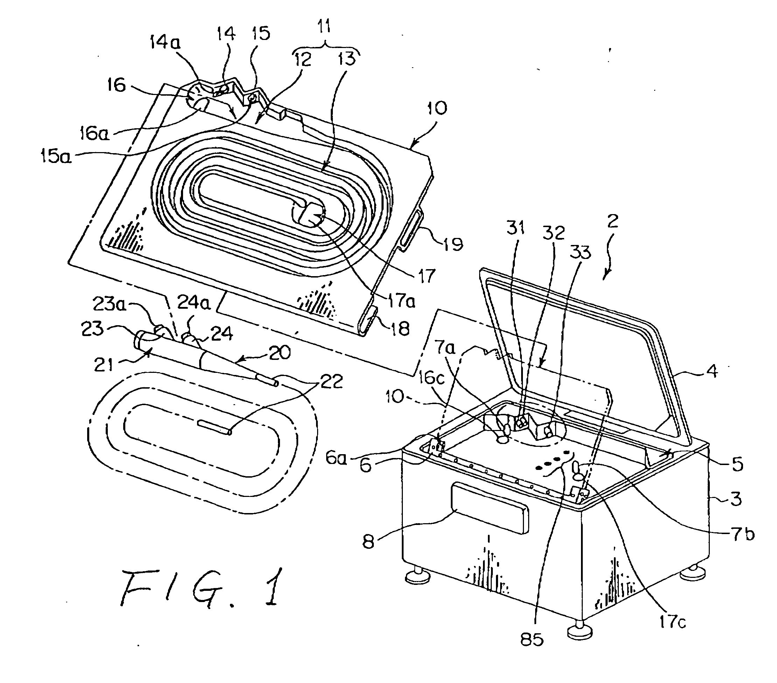

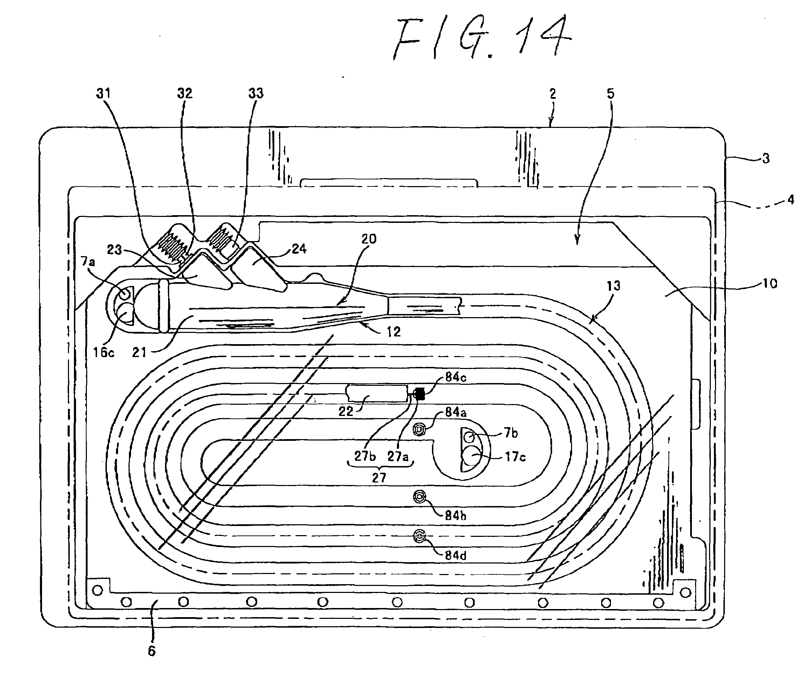

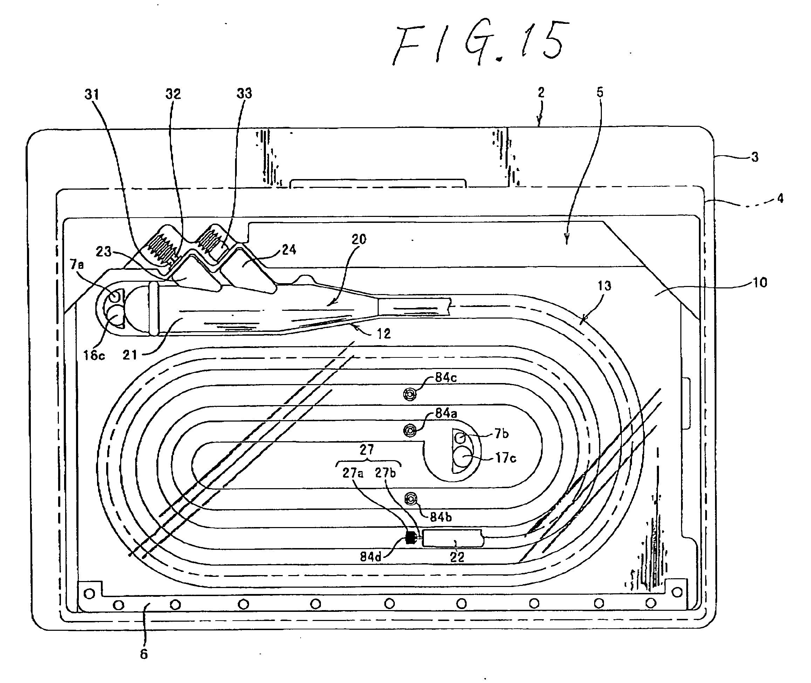

Referring to Figs. 1 to 15 and 18, a first embodiment of an endoscope washing and disinfecting apparatus of the present invention will now be described.

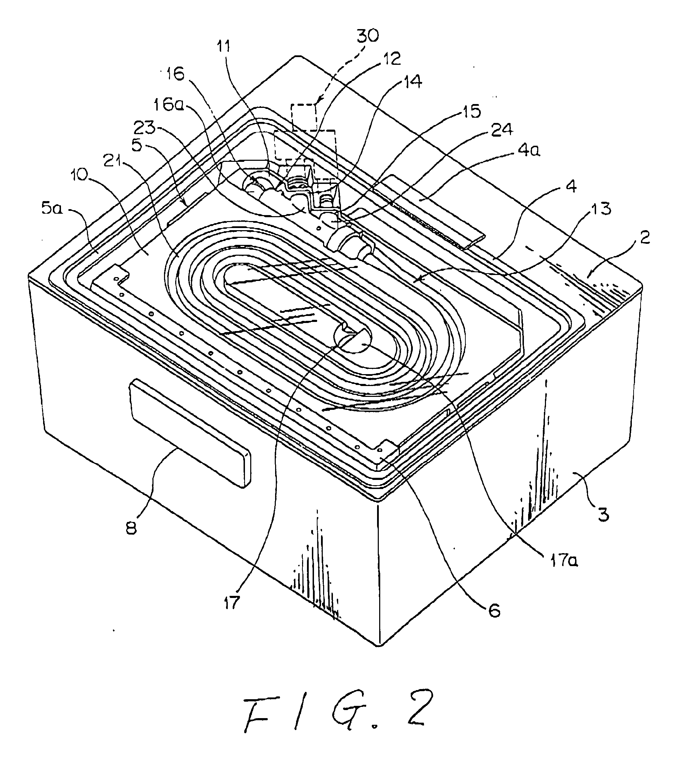

Figs. 1 and 2 illustrate the overall configuration of an endoscope washing and disinfecting apparatus 2 according to the present embodiment.

This endoscope washing and disinfecting apparatus 2 is provided with a main body unit 3, a top cover 4, and a tray 10. The main body unit 3 has a washing bath 5 of a predetermined depth at the top of the main body unit 3. The top cover 4 is disposed to cover the opening of the washing bath 5.

A tray holder 6 is rotatably disposed at an edge of the washing bath 5. A tray 10 for holding a used endoscope (which is simply a tray 10), which provide an endoscope loading plane, is detachably disposed at the tray holder 6. The washing bath has a bottom, on which there are disposed first and second protrusion 7a and 7b.

A water supply port 16c is formed in the vicinity of the first protrusion 7a, while a drain port 17c are formed in the vicinity of the second protrusion 7b. Washing fluid and rinsing water is supplied from the water supply port 16c to both the washing bath 5 and the tray 10. The fluid fed from the water supply port 16c is discharged outside the washing bath 5 via the drain port 17c.

On the front of the main body unit 3, there is a formed an operation panel 8 with which an operator is able to give various data to the apparatus 2 and receive various information from the apparatus 2 in an interactive manner.

The top cover 4, made of hard and light-transmittance resin material, that is, transparent or semitransparent resin material, is formed into a predetermined shape and disposed at an edge of the washing bath 5 to allow the top cover 4 to be open and closed. Hence, even if the top cover 4 is closed to cover the opening of the washing bath 5, an operator can visually observe the inside of the washing bath 5 through the top cover 5.

Incidentally, the endoscope washing and disinfecting apparatus 2 according to the present embodiment is able to cope with washing and disinfecting, besides the endoscopes, other medical tools, such as therapeutic instruments with ducts or tubular through-holes and over tubes. For washing and disinfecting those medical tools, trays dedicated to hold such tools are prepared.

In the present embodiment, an endoscope 20 is mounted on the tray 10 and accommodated in the washing bath 5. This tray 10 is produced as a holding plate dedicated to this endoscope 20 itself. If the type of an endoscope being washed differs from that of this endoscope 20, another tray produced dedicatededly to the endoscope is used.

As shown in Fig. 1, the present endoscope washing and disinfecting apparatus 2 further comprises washing nozzles 31, 32 and 33 on a side wall near a corner of the washing bath 5. These washing nozzles 31, 32 and 33 are members connected with or disconnected from various channels (ducts) of an endoscope 20 for washing and disinfecting them and compose one of main parts of a nozzle-mounted device 30 arranged in the main body unit 3, as will be described later.

The tray 10 is used to mount a used endoscope 20 thereon. The tray holder 6 has a holding member 6a to hold the tray 10, as shown by a chain double-dashed line in Fig. 1.

The endoscope 20 is provided with a base portion 21 and an elongated long insertion tube 22 extended from the base portion 21. This insertion tube 22 is flexible. The base portion 21 comprises an air/water-supply channel connector 23 and a suction channel connector 24, which are obliquely protruded from a side of the base portion 21 to form an acute angle from the base end of the base portion 21. Both connectors 23 and 24 are parallel to each other.

Of these connectors 23 and 24, the air/water-supply channel connector 23 has air/water-supply connecting ports 23a, which are connected to both an air-supply connecting member, to which one end of an air-supply duct is connected, and a water-supply connecting member, to which one end of a water-supply duct is connected, respectively. Meanwhile the suction channel connector 24 has a suction connecting port 24a, which is connected to a suction connecting member, to which one end of a suction channel (duct) 22a is connected. Thus, during an examination with the endoscope 20, the air/water-supply connecting ports 23a are connected with air/water-supply tubes (not shown) to supply air and water to the endoscope 20. Meanwhile, the suction connecting port 24a is connected with a suction tube (not shown) arranged through the insertion tube 22.

The base portion 21 is used as a gripping portion which is gripped by a user who desires to operate the endoscope 20. In the case of fixedly securing the endoscope 20 to fixing members such as arms, this base portion 21 is used as a fixing member.
On the tray 10 is formed a accommodating groove 11 (a kind of recess) which serves as a guide groove to allow the endoscope 20 to be located and accommodated at predetermined positions on the tray. The accommodating groove 11 has a predetermined contour formed in accordance with the outer shapes of both the base portion 21 and the insertion tube 22. This means that the tray 10 is produced to be dedicated to each endoscope 20, type by type, of which accommodating groove 11 is fit to the outer shape of each endoscope 20. If there are plural types of endoscopes in a medical facility (i.e., such endoscopes have base portions and/or insertion tubes having different outer shapes and/or lengths), a plurality of types of trays 10 dedicated to those various type endoscopes may be prepared.

To be specific, the accommodating groove 11 consists of a base-accommodating recess (groove) 12 into which the base 12 of the endoscope 20 is placed for accommodation and an almost vortical tube-accommodating groove 13 into which the insertion tube 22 thereof is placed for accommodation. The tube-accommodating groove 13 is formed into a substantially vortical shape on the tray 10. The recess 12 and the groove are formed as a continuous groove for guiding the placement of the endoscope 20 onto the tray 10.

The base-accommodating recess 12 is provided with, as part of the side wall thereof, an air/water-supply channel accepting member 14 and a suction channel accepting member 15 at both of which the air/water-supply channel connector 23 and the suction channel connector 24 are disposed, respectively. Of these members 14 and 15, the air/water-supply channel accepting member 14 has an opening 14a through which the air/water-supply connecting ports 23a is secured. On the other hand, the suction channel accepting member 15 has an opening 15a through which the suction connecting port 24a is secured.

At a predetermined position in base of the base-accommodating recess 12, a first water port 16 is formed to feed and discharge water such as washing water and disinfecting water. Similarly, at a predetermined position in the base of the tube-accommodating groove 13, a second water port 17 is formed to feed and discharge water such as washing water and disinfecting water. The first water port 16 is positioned in proximity to the base side of the base portion 21. The second water port 17 is positioned in proximity with a distal surface of the insertion tube 21. Each of the first and second water ports 16 and 17 has a lid 16a (17a) which can be opened and closed. The lids 16a and 17a are held to always close the water ports 16 and 17 by not-shown forcing members combined with their weight. Only the weight of each lid 16a (17a) can be utilized as such forcing means. With no tray placed in the washing bath 5, the lids 16a and 17a are made to close. Thus, when an endoscope 20, which has been used so far, is accommodated in the accommodating groove 11, humor and/or filth on the endoscope 20 is prevented from leaking out through the first and second ports 16 and 17. Therefore, with the endoscope 20 accommodated in the accommodating groove 11 of the tray 10, the endoscope 20 can be carried sanitarily.

In the present embodiment, the first water port 16 is used to feed washing water, disinfecting water, and so on into the accommodating groove 11 therethrough. And, through the second water port 17, the water such as washing water and disinfecting water, which has been fed in the accommodating groove 11, is drained from the groove 11 to the washing base 5. In order to allow the base portion 21 and insertion tube 22 of an endoscope 20 accommodated in the accommodating groove 11 to be immersed sufficiently in the washing of disinfecting water supplied through the first water port 16, the accommodating groove 11 is formed so that there is no useless space left between the endoscope 20 and the wall of the accommodating groove 11. It is therefore possible to minimize amounts of washing water and disinfecting water required.

As shown in Fig. 1, the tray 10 has an attachment portion 18 which is used to attach the tray 10 to the holding member 16a at the longitudinal edge of the washing bath 5. The attachment portion 18 is shaped into for example a U-shaped form, which is to be adaptable to the holding member 6a. A reference 19 in Fig. 1 shows one of gripping hands for carry. The gripping hands 19 are located on both lateral side edges of the tray 10. To avoid interference with the top cover 4, the gripping hands 19 are made to protrude downward from the tray 10.

When an endoscope 20 is accommodated in the accommodating groove 11 on the tray 10, both connecting ports 23a and 24a of the air/water-supply channel connector 23 and suction channel connector 24 of the endoscope 20 are fixedly placed through the opening 14a of the air/water-supply channel accepting member 14 and the opening 15a of the suction channel accepting member 15, respectively. By this placement, the used endoscope 20 can easily be accommodated in the accommodating groove 11 with a predetermined attitude thereof.

After accommodating the endoscope 20 in the accommodating groove 11, the tray 10 is made to be connected with the tray holder 6, as shown in Fig. 2. A proper attachment of the attachment portion 18 of the tray 10 with the holding member 6a of the tray holder 6 makes it possible that the tray 10 is rotated in the downward by hand or with an automatic mechanism (not shown). Thus the tray 10 can be put into the washing bath 5.

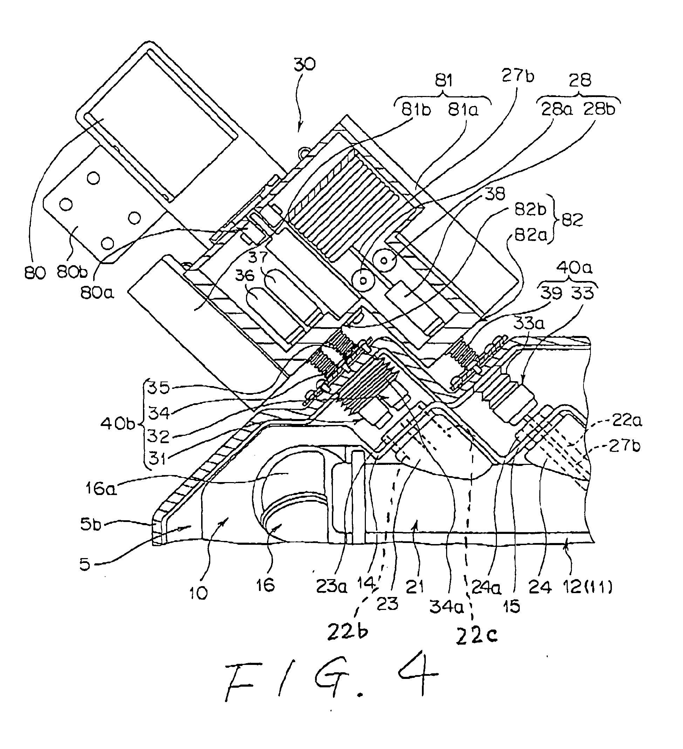

In response to the downward rotation of the tray 10, the first and second protrusions 7a and 7b on the base of the washing bath 5 push up the lids 16a and 17a so as to make the first and second water ports 16 and 17 open, respectively. Concurrently with this open actions of the lids 16a and 17a, as shown in Fig. 4, both connecting ports 23a and 24a of the endoscope 20, which are placed to protrude from the openings 14a and 15b of the side wall of the accommodating groove 11, are located to face an air-supply channel washing nozzle 31, a water-supply channel washing nozzle 32, and a suction channel washing nozzle 33, respectively, with a predetermined distance apart from the nozzles.

When the accommodation of the endoscope 20 is completed, the top cover 4 is moved by hand or with the use of an automatic mechanism (not shown), whereby the washing bath 5 is covered as shown in Fig. 2.

A packing 5a is attached on the upper surface of the main body unit 3 in such a manner that the packing 5a surrounds the edge of the washing bath 5. Hence when the top cover 4 closes the washing bath 5, the packing 5a is pressed down by the top cover 4 so as to sustain the watertight performance between the top cover 4 and the washing bath 5. This watertight performance prevents the liquid within the washing bath 5 from scattering outside the main body unit 3. A hinge 4a is secured on an edge of the top cover 4 for opening and closing the top cover 4.

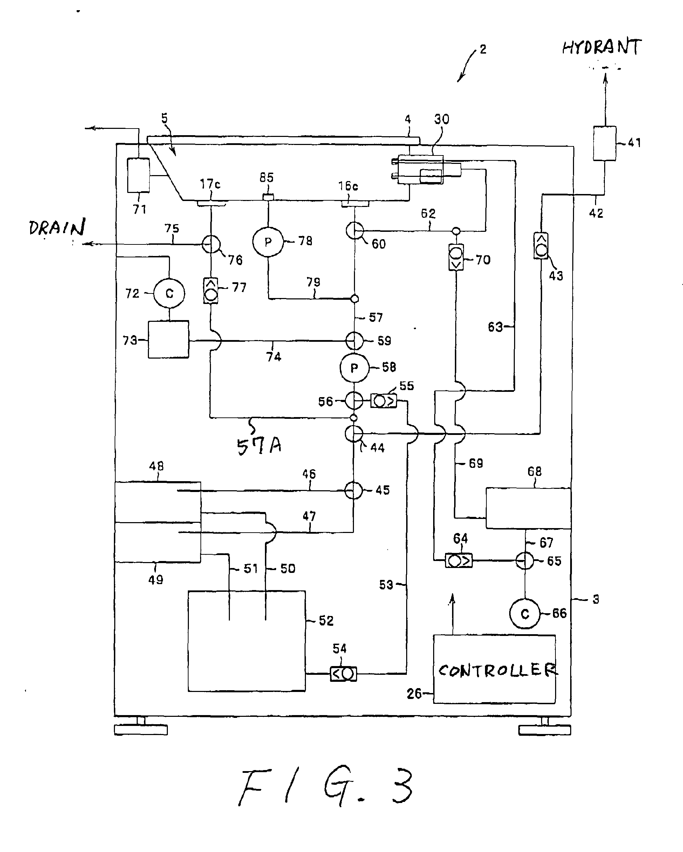

Referring to Fig. 3, the piping structure of the endoscope washing and disinfecting apparatus 2 will now be described.

As illustrated in Fig. 3, the main body unit 3 of this apparatus 2 introduces a duct 42 connected to a hydrant, so that the tap water comes into the unit 3 through the duct 42. Along this duct 42, a water filter 42, a check valve 43, and tow three-way switching valves 44 and 45 intervenes in this order from the hydrant side. In addition, this duct 42 is branched into two branch ducts 46t and 47 at the one three-way switching valve 45.

Of these two branch ducts 46, one duct 46 is linked with a washing agent bottle 38, while the remaining duct 47 is linked with a chemical bottle 49. A duct 50 connects the washing agent bottle 38 and a stirring bath 52 and another duct 51 connects the chemical bottle 49 and the stirring bath 52, with the result that a washing agent or a chemical flows into the stirring bath 52 from the respective bottles 48 and 49.

Thus the tap water from the hydrant first passes the water filter 41 for filtering. The filtered tap water then passes the ducts, and then is branched into both ducts 46 and 47 to flow into the washing agent bottle 48 or the chemical bottle 49. The washing agent in the bottle 48 or the disinfectant in the bottle 49 is diluted with the tap water to become a solution of a predetermined concentration. This solution is therefore fed to the stirring bath 52 via the connecting duct 50 or 51.

Each of the three-way switching valves 44 and 45 has an internal fluid path which is switched from one type to another depending on electrical command signals issued by a controller 26 which works on what the current process is, i.e., a washing process, a disinfecting process, or a rinsing process. By way of practical example, for the rinsing process, the internal path of the three-way switching valve 44 is switched so as to connect the duct 42 to another circulating duct 57. Responsively to this switch, the tap water is guided to pass through the duct 57, which allows the water to be used for washing the outer surface and the various endoscopic ducts.

To the stirring bath 52 is connected one end of a feeding duct 53 for transferring liquid. The other end of this feeding duct 53 is linked with the circulating duct 57 via the three-way switching valve 56. Check valves 54 and 55 are inserted in the feeding duct 53. The circulating duct 57 connects both water supply ports 16c and 17c of the washing bath 57. Between the three-way switching valve 56 and the water supply port 16c in this duct 57, a circulating pump 58 and two three-way switching valves 59 and 60 are inserted, as shown in Fig. 3.

Connected to the three-way switching valve 60 is one end of a duct 62 to lead to the endoscopic channels. The other end of this duct 62 is branched into two paths and linked with the nozzle-mounted device 30.

When the circulating pump 58 is driven under the control of the controller 26, the washing fluid or disinfecting fluid in the stirring bath 52 passes into the circulating duct 57 via the feeding duct 53 and the three-way switching valve 56. The path of this fluid is selected into the path to the water-supply port 16c or the duct 62 for the endoscopic channels by the three-way switching valve 60 under the control of the controller 62. Thus, the outer surface and the various channels of the endoscope 20 are subjected to washing and disinfection, and the fluid which has been used for the washing and disinfection is reserved in the washing bath 5.

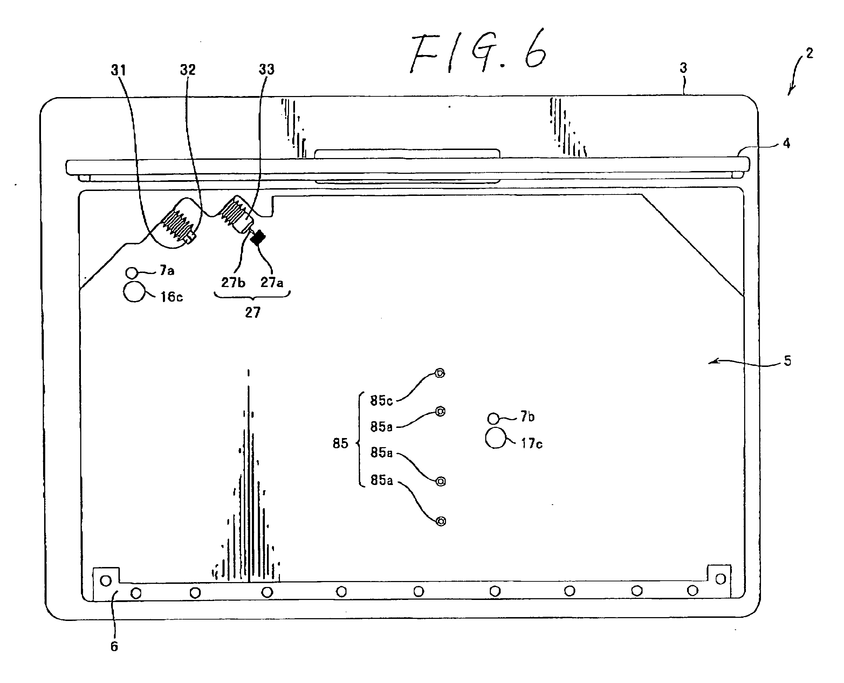

In addition, one end of a duct 57 for washing a brush 27 (refer to Fig. 6) is connected to the circulating duct 57 between the two three-way switching valves 59 and 60. The brush 27 is used for washing endoscopic channels, such as a suction channel, formed through the insertion tube 22 of the endoscope 20. In the course of this duct 79, there is provided a pump 78 for washing the brush 27, and the remaining end of this duct 79 is lined with nozzle connectors 85 disposed at the base of the washing bath 5. In response to drive of the pump 78 which is carried out under the control of the controller 26, the washing fluid, disinfecting fluid, or tap water in the circulating duct 57 is controlled to flow to the nozzle connectors 85 depending on a commanded process.

The drain port 17c is connected to the circulating duct 57 between the three-way switching valves 56 and 44 through a duct 57A in which there are inserted a three-way switching valve 76 and a check valve 77 in this order from the drain port 17c. The three-way switching valve 76 is also connected with a drain duct 75 and the internal path of this valve 76 is switched in a controlled manner under the controller 26. Hence it is possible that the fluid that remains in the washing bath 5 is drained to an outside drain port via the drain port 17c, the three-way switching valve 76 in the duct 57A, and the drain duct 75.

The three-way switching valve 59 is also connected to one end of an air-supply duct 74 in which a compressor 72 and an air filter 73 are placed. The air supplied from the compressor 72 is fed, under the control of the controller 26, to both the water-supply port 16c and the nozzle-mounted device 30 for dehydrating water droplets and moisture on the outer surface of the washed and disinfected endoscope 20 and inside the various channels thereof.

The nozzle-mounted device 30 is also linked with an end of a water-leakage sensing duct 63 in which there are placed a check valve 64 and a three-way switching valve 65. The remaining end of this duct 63 is linked with another compressor 66 used for sensing water leakage.

The three-way switching valve 65 Is connected to one end of a duct whose other end is connected to an alcohol tank 68. Also connected to this tank 68 is one end of a duct 69, of which other end is connected to the duct 62 via a check valve 70 inserted in the duct 69.

The compressor 66 operates to supply air to the duct 63 in response to commands from the controller 26 in order to sense water leakage from the endoscope 20 or to supply the alcohol in the tank 68 to the ducts 69 and 62 in order to apply alcohol flush to the various channels of the endoscope 20.

Furthermore, the washing bath 5 is formed to communicate with the outside via a deodorant filter 71 to remove abnormal odor in the bath 5.

Referring to Fig. 4, the nozzle-mounted device 30 equipped with the nozzles 31, 32 and 33 for washing the endoscopic channels will now be detailed.

As shown in the figure, the nozzle-mounted device 30 is a device which automatically connects or disconnect the nozzles 31, 32 and 33 to or from the opening of the channels formed through the insertion through the endoscope 20. When this connecting and disconnecting operation is carried out, the endoscope 20 is mounted in the accommodating groove 11 on the tray 20.

More concretely, only one action makes it possible that the opening of the suction channel 22a (which opens at the suction connecting port 24a) is automatically connected with the nozzle 33 for washing the suction channel 22a mounted in the device 30 and both openings of the air-supply channel 22b (refer to Fig. 4) and water-supply duct 22c (refer to Fig. 4) (which open at air/water-supply connecting ports 23a) are automatically connected with both nozzles 31 and 32 for washing the air-supply duct and water-supply duct, respectively. In addition, another only one action allows those connected three nozzles 31-33 to be released from the openings of the channels. For realizing those connection and disconnection actions, the nozzle-mounted device 30 is disposed to have a predetermined positional relationship to the base-accommodating recess 12 of the tray 10.

The suction channel 22a, air-supply channel 22b, and water-supply channel 22c are endoscopic ducts which are formed to extend from the base portion 21 to the insertion tube 22 and open at the distal end surface of the insertion tube 22.

The nozzle-mounted device 30 is equipped with, as its essential components, a suction-side connecting part 40a, an air/water supply-side connecting part 40b, a pair of rail members 81a and 81b composing guide means 81, a latch type solenoid 80, and a nozzle-mounted block 82. The nozzle-mounted block 82 is a void box-shaped member composed of a suction-side block 82a to which the suction-side connecting part 40a is connected and an air/water supply-side block 82b to which the air/water supply-side connecting part 40b is connected.

The rail members 81a and 81b are fixed disposed to be parallel to each other and to position to provide a predetermined guide structure to the main body unit 3. The nozzle-mounted block 82 is arranged slidably between the mutually parallel rail members 81a and 81b, so that this block 82 can be moved in both directions consisting of a direction advancing toward a washing-bath frame 5b and a direction opposite to the advancing direction, that is, a direction retreating the frame 5b.

The nozzle-mounted block 82 has sliding surfaces to be touched to the rail members 81a and 81b, a solenoid-fixing surface on which the solenoid 80 is fixed, and a nozzle-fixing surface with a step portion through which the duct-washing nozzles 31, 32 and 33 are fixedly disposed. The latch type solenoid 80 has a solenoid shaft 80a of which distal end is fixedly secured to the solenoid-fixing surface. On the back side of the nozzle-mounted block 82, plural connecting springs (not shown) are placed to couple their one ends to the back. The other ends of those connecting springs are coupled to either of the rail members 81a and 81b.

The latch type solenoid 80 has a securing plate 81b, which is secured at a predetermined position of the main body unit 3. In the present embodiment, the securing position of this solenoid 80 is decided such that, in cases where this endoscope washing and disinfecting apparatus 2 is in a standby for washing and disinfection, the solenoid shaft 80a is forced to be pulled in the solenoid by its magnetic force so that the nozzle-mounted block 82 is forcibly attracted to a predetermined position near to the solenoid 80.

That is, for sustaining the nozzle-mounted block 82 at the predetermined position near the solenoid 80, the magnetic force caused in the solenoid 80 is balanced with the pushing force of the elastically deformed connection springs (not shown). When the magnetic force is diminished in the solenoid, the pushing force of the connection springs moves the nozzle-mounted block 82 toward the washing-bath frame 5b.

The suction-side connecting part 40a is provided with the foregoing washing nozzle 33 for the suction channel 22a, a buffer spring 39, and an L-shaped pipe 38. Meanwhile the air/water supply-side connecting part 40b is provided with the foregoing washing nozzle 31 for the air-supply channel 22b, the forgoing washing nozzle 32 for the water-supply channel 22c, an buffer spring 34, a buffer spring 35, and L-shaped pipes 36 and 37.

All the channel washing nozzles 31, 32 and 33 are fixedly secured to the nozzle-mounted block 82, respectively, and have longitudinal axes which are parallel to each other along the same plane.

Of these nozzles 31 to 33, the suction-channel washing nozzle 33 is located such that the nozzle 33 protrudes, by a predetermined length, from the front (nozzle fixing surface) of the washing bath frame 5b in front of the suction-side block 82a. Further, the air/water-supply channel washing nozzles 31 and 32 are located such that both nozzles 31 and 32 protrude, by a predetermined length, from the front (nozzle fixing surface) of the washing bath frame 5b in front of the air/water-supply side block 82b.

These nozzles 31 to 33 are placed to make their distal ends protrude in the washing bath 5 through through-holes (not shown) formed through the washing-bath frame 5b. The suction-side connecting part 40a still has a watertight sustaining member 33a, made of elastic material, having a cover portion and a folded portion both covering the nozzle 33, while the air/water supply-side connecting part 40b still has a watertight sustaining member 34a, made of elastic material, having a cover portion and a folded portion both covering both nozzles 31 and 32.

The buffer springs 39, 34 and 35 are loaded to the washing nozzles 33, 31 and 32, respectively, between the nozzle-mounted block 82 and the washing-bath frame 5b. These buffer springs 39, 34 and 35 are employed to absorb shocks caused by inserting each nozzle into each opening of each connecting port(s) 24a (23a) of the base portion 21 of the endoscope 20.

Meanwhile, with the base ends of the nozzles 31, 32 and 33, the L-shaped pipes 36, 37 and 38 are fixedly coupled. Though not shown, these pipes 36 to 38 are connected with one ends of an air-supply tube, a water-supply tube, and a sucking tube on the back side of the device 30, respectively. The other ends of these tubes are connected to the duct 62 for the endoscopic channels within the main body unit 3.

The suction-side block 82a still accommodates therein a brush device 27 essentially consisting of a brush 27a and a brush wire 27b (refer to Fig. 6). The brush device 27 is exchangeable. The brush wire 27b is accommodated in a wound form. The brush wire 27b is pressed between two rollers 28a and 28b, which compose a pair of rollers placed in the suction-side block 82a, and extend through the L-shaped pipe 38 and the connecting part 40a in an airtight manner.

Of the two rollers, one is a driving roller 28a and the other is a driven roller 28b. The driving roller 28a has a shaft (not shown) driven by a rotating drive force coming from a motor via a reduction gear train (not shown). Thus the brush wire 27b is forced by the rotation of the driving roller 28a so as to advance or retreat so that the brush 27a is inserted from the suction-side block 82a into the suction channel 22a via the connecting part 40a or pulled back from the suction channel 22a of the endoscope 20 to the suction-side block 82a via the connecting part 40a.

As explained above, in the endoscope washing and disinfecting apparatus 2, the washing nozzles 31, 32 and 33 are used to supply washing fluid, disinfecting fluid rinsing water, and/or deodorizing air to the suction channel 22a, air-supply channel 22b, and water-supply channel 22c in a controlled manner depending on each of the washing, disinfecting, rinsing, and/or deodorizing steps. By this supply, the endoscope 20 is subjected to washing and disinfection of the outer surface and inner channels of the endoscope 20.

Further, in the endoscope washing and disinfecting apparatus 2, the suction channel 22a of the endoscope 20 is subjected to brushing by the brush 27a which is made to go back and forth, in addition to supply of the washing fluid, disinfecting fluid, or rinsing water. Hence this apparatus 2 is able to wash and disinfect the suction channel 22a by removing, in particular, various kinds of humor, such as mucosa and blood, and filth attached thereon in a reliable and consistent manner.

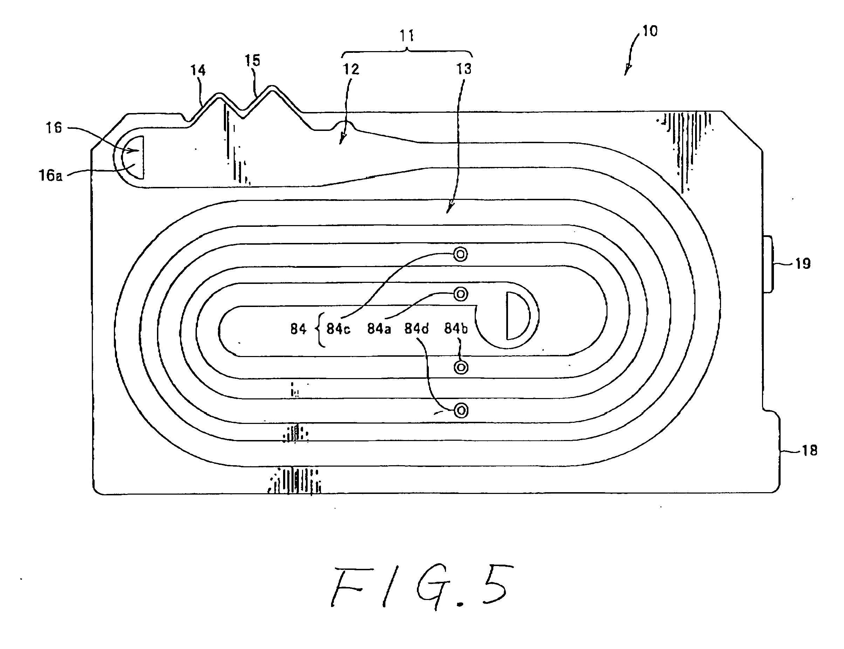

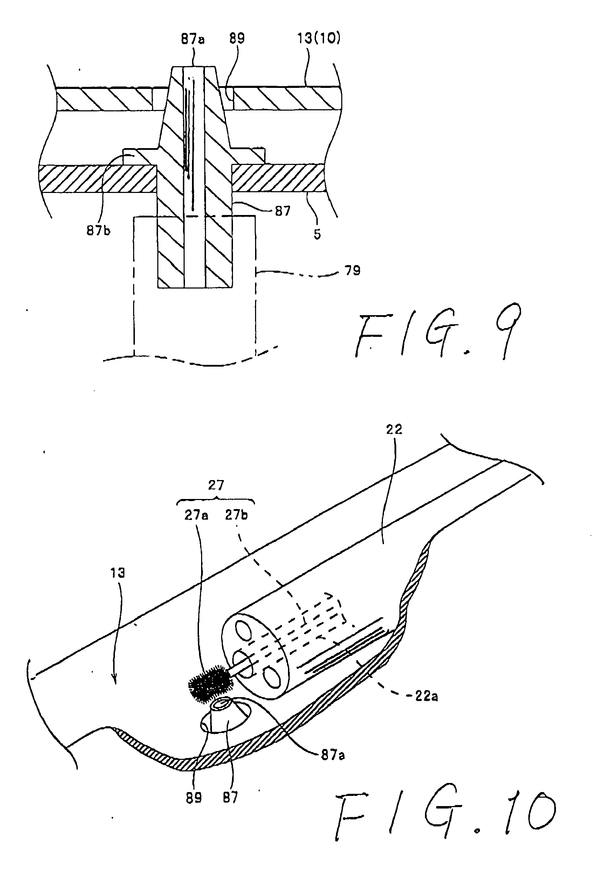

Referring to Figs. 5 and 8, another configuration provided by the present endoscope washing and disinfecting apparatus 2 will now be described. This configuration relates to washing the brush 27a while the brush 27a is made to repeat the advancing and retreating actions along the suction channel 22a.

Specifically, as shown in Fig. 5, the tray 10 is provided with a plurality of brush washing nozzles 84 (four nozzles 84a, 84b, 84c and 84d in the present embodiment) which function as fluid spraying members. These nozzles 84 are positioned at four different positions along the tube-accommodating groove 13, which is part of the accommodating groove 11 for the used endoscope 20. The four washing nozzles 84a, 84b, 84c and 84d are dependent on the types of endoscopes to be washed, specifically, on the lengths of insertion tubes of those four different endoscopes. The length is defined as a longitudinal (length-wise) length of each insertion tube. The positions of the four nozzles 84a, 84b, 84c and 84d are consistent with those of the distal ends of insertion tubes being accommodated.

To be more specific, the bush washing nozzle 84a located an innermost position on the tray 10 is prepared for an endoscope 20 having a longest insertion tube 22 and is formed at a longitudinal position in proximity to the distal end thereof mounted on the base of the tube-accommodating groove 13. In contrast, the bush washing nozzle 84a located an outermost position on the tray 10 is prepared for an endoscope 20 having a shortest insertion tube 22 and is formed at a longitudinal position in proximity to the distal end thereof mounted on the base of the tube-accommodating groove 13.

The remaining two brush washing nozzles 84b and 84c located intermediate positions on the tray 10 are prepared for endoscopes 20 having intermediate insertion tubes 22. And each of these nozzles 84b and 84c is also formed at a longitudinal position in proximity to the distal end thereof mounted on the base of the tube-accommodating groove 13.

In terms of the lengths of the insertion tubes 22 (provided that the sizes of the endoscopes 20 are the same), the four exemplified nozzles 84a, 84b, 84c and 84d are set realize the relationship of 84a>84d>84c>84d which are longer in this order.

In addition, as shown in Fig. 6, in the base of the washing bath 5, there are disposed four nozzle connectors 85 consisting of members 85a, 85b, 85c and 85d, of which positions are decided to be in agreement of the horizontal positions of the four brush washing nozzles 84a, 84b, 84c and 84d on the tray 10. Thus, when the tray 10 is installed in the washing bath 5, a mechanical connection is realized between the nozzle connector 85a and the washing nozzle 84a, between the nozzle connector 85b and the washing nozzle 84b, between the nozzle connector 85c and the washing nozzle 84c, and between the nozzle connector 85d and the washing nozzle 84d, respectively.

As a modification, the number of brush washing nozzles 84 may not be limited to four, but may be one, two, three, or five or more. The number of nozzle connectors 85 may be set corresponding to the number of nozzles 84 to be set.

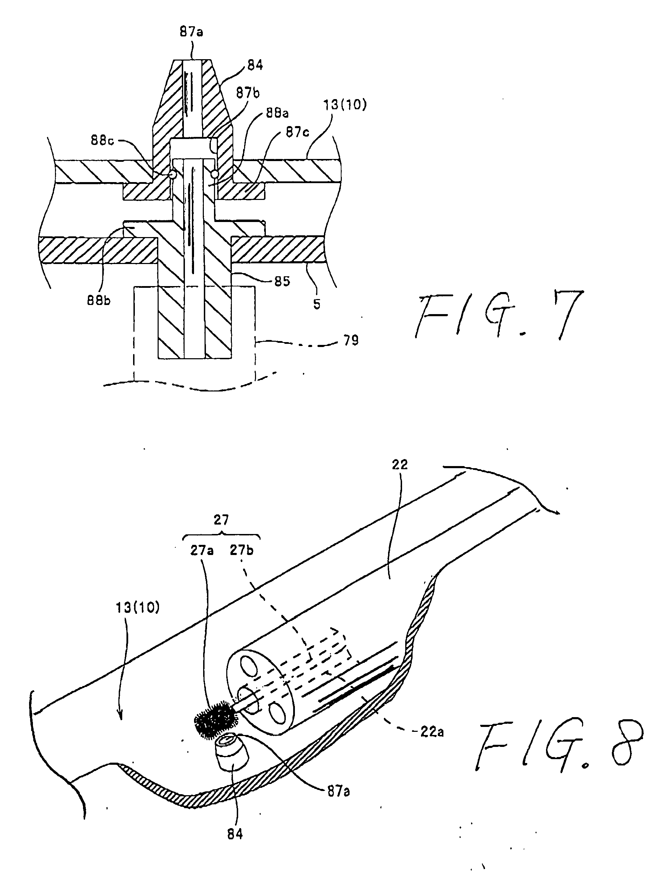

As illustrated in Fig. 7, each brush washing nozzle 84 is shaped into a substantial tube with a tapered outer surface on the top side thereof. This nozzle 84 has a through-hole formed inside through a longitudinal direction thereof, the through-hole providing a spray opening 87a which opens at the top side and a fitting hole 87b into which the nozzle connector 85 is fit. The base of each nozzle 84 is widened in diameter to form a flange 87c. Each flange 87c is made to touch the back surface of the tube-accommodating groove 13 when the connectors 85 are fit into the nozzles 84.

Each nozzle connector 85 is also shaped into a substantial tubular member, but it has, at an upper side thereof, a fit portion 88a being fit into the fitting hole 87b of each nozzle 84. A O-ring 88c is placed on the outer surface of the fit portion 88a so as to keep the airtight performance with the fitting hole 87b. The connector 85 still has a flange 88b formed to extend at a longitudinal intermediate position thereof. The nozzle connector 85 is secured to the base of the washing bath 5 so that the flange 88b is water-tightly mounted on the upper surface of that base. The lower end portion of each connector 85 is coupled with the foregoing duct 79.

As stated, a used endoscope 20 is accommodated in the accommodating groove 11 on the tray 10, and then the tray 10 is installed in the washing bath 5. In this installed state, the work processes necessary for washing and disinfecting the accommodated endoscope 20 are carried out. By way of example, in each of these work processes, the brush wire 27b, that is, the brush 27a, is mechanically driven to repeatedly advance and retreat along the suction channel 22a in response to commands from the controller 26. During such repeated actions, as illustrated in Fig. 8, the brush 27a appears form the frontal surface of the insertion tube 22. Namely the actions of the brush 27 are controlled such that the brush 27 protrudes from the front by a predetermined length. Setting is made such that, when protruding to the fullest extent, the brush 27b is located at a position just above a specific nozzle 84. At this moment and in this fullest protruded stage, the fluid for washing, disinfecting and rinsing is forcibly sprayed from the spray opening 87a of the nozzle 84 upward to the brush 27a.

The washing process for the brush 27a may be carried out only during or after the washing process for the suction channel 22a.

Thanks to this spray action, the various kinds of humor, such as mucosa and blood, and filth attached to the brush 27a are removed. In addition, in parallel to washing the brush 27a, the washing, disinfesting, or rinsing fluid is controlled to flow through the suction channel 22a without rest, so that a flow of the fluid from the distal opening of the suction channel 22a is also kept during washing the brush 27a. As a consequence, such flow surely prevents the contaminated articles, once removed from the brush 27a, from penetrating the suction channel 22a from its distal opening.

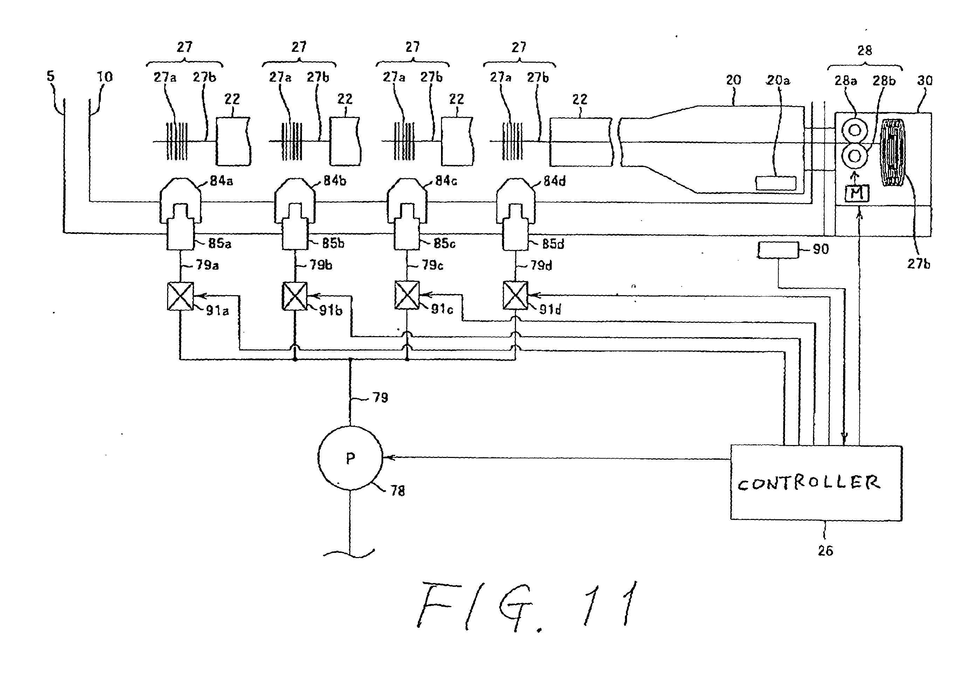

A modification is shown in Figs. 9 and 10, in which the brush washing nozzle 87 is disposed at a predetermined position on the base in the washing base 5, so that its spray opening 87a is directly protruded from the base of the tray 10. To accomplish this modification, a through-hole that allows the insertion of the nozzle 87 is formed through the tube-accommodating tube 13. The lower end portion of the nozzle 87 is linked with the duct 79 in the same way as explained before. Thus the nozzle 87 can be made in a simpler manner.

It is preferred that the endoscope washing and disinfecting apparatus 2 is of a universal type. That is, the apparatus 2 is adaptive by itself to washing various kinds of endoscopes 20 with various-length insertion tubes 22. In order to attain such an object, the brush washing nozzles 84a to 84d is should be driven selectively depending on the lengths of the insertion tubes 22, that is, the kinds of the endoscopes 20. Referring to Figs. 11-15 and 18, the configurations and operations for such an object will now be described.

As pictorially shown in Fig. 11, depending on the number of nozzle connectors 85, the duct 79 coming from the pump 78 is divided into first to fourth ducts 79a to 79b connecting to electromagnetic valves 91a to 91d, respectively.

The respective electromagnetic valves 91a to 91d are responsive to control signals from the controller 26 and their internal valve members can be opened and closed by such control signals. The controller 26 is communicably connected to a receiver 90, which receives endoscopic information about the endoscope 20 by wireless from an endoscope ID (identification) medium 20a attached to the endoscope 20. The endoscopic information includes data showing the types of various optical systems, the length of an insertion tube, and the diameters of various endoscopic channels (ducts), which are inherently given to the endoscope accommodated in the accommodating groove 11. The receiver 90 is formed to magnetically or optically read the information from the ID medium 20a, which is for example an IC chip or a bar code.

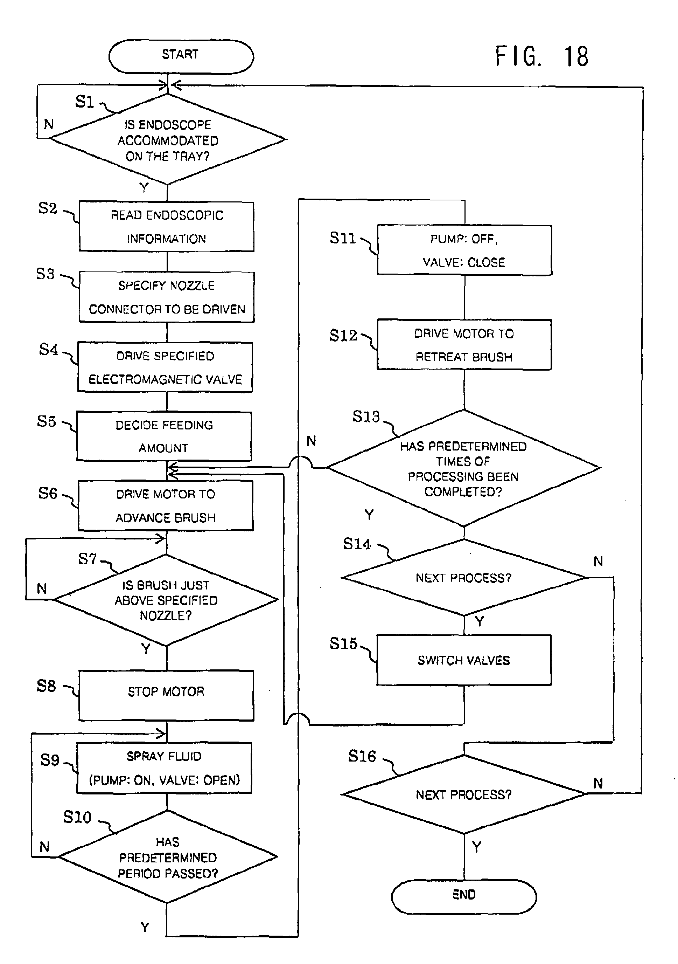

The controller 26 first receives operation information from the operation panel 8 to determine whether or not an endoscope 20 being washed and disinfected is accommodated in the groove 11 on the tray 10 (step S1 in Fig. 18). And the controller 26 reads out, by wireless, the endoscopic information from the endoscope ID medium on the endoscope 20 (step S2).

The controller then shifts the next processing to interpret the length of the insertion tube 22 from the read-out endoscopic information, to use the interpreted length information to select an appropriate brush washing nozzle 84 adaptive to the length of the insertion tube 22 of the endoscope 20 currently accommodated, and to specify a nozzle connector 85 connected to the selected nozzle 84 (step S3).

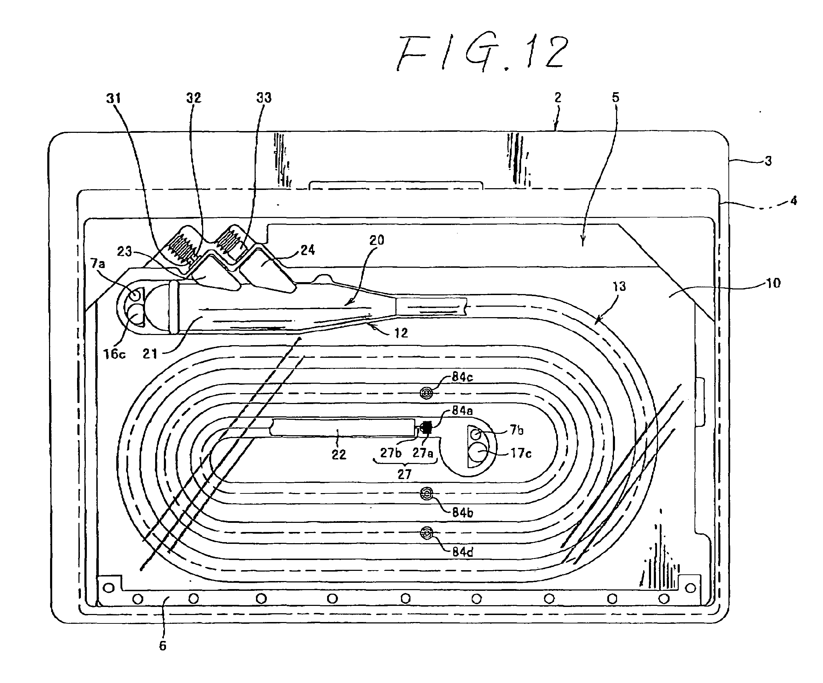

Fig. 12 exemplifies this situation in more detail. As shown, an endoscope 20 which uses the longest insertion tube 20 is accommodated in the base-accommodating recess 12 and tube-accommodating groove 13, the controller 26 specifies the nozzle connector 85a connected to the brash washing nozzle 84a of which position in the groove 13 matches with the length of the insertion tube 12, on the basis of the read-out length information from the endoscope ID medium 20a.

Hence the controller 26 controls only the electrometric valve 91a in the first branched duct 97a connected to the nozzle connector 85a in such a manner that the valve 91a is opened (step S4). During this control, the remaining electromagnetic valves 91b to 91d, which are in the second to fourth branched ducts 79b to 79d connected to the other nozzle connectors 85b to 85d, are kept at their valve-closed states (i.e., initial states).

From the read-out length information about the insertion tube 20, the controller 26 decides a feeding amount to insert and pull back the brush 27a through the suction channel 22a (that is, an mount on which the brush wire 27b is fed and pulled back by the rollers 28 in the nozzle-mounted device 30) (step S5). In this decision, the controller 26 sets the feeding amount (in the advancing direction along the duct 22a) at its maximum value which allows the brush 27a to just be located above the spray opening 87a of the nozzle 84a. Then in reply to the decided feeding amount for the insertion tube 22, the controller 26 drives a motor M to rotate the roller 28 (step S 6).

In addition, the controller 26 commands the motor M to stop for a predetermined period of time (for example, several seconds) to stop the rotation of the roller 28, when the brush 27a which has advanced, to the fullest extent, to protrude from the frontal surface of the insertion tube 22 is just above the nozzle 84a (steps S7 and S8).

During this stop period, the controller 26 orders the rotational drive of the pump 78 and the open of the electromagnetic valve 85a, so that the washing, disinfecting, or rinsing fluid is forcibly sprayed up toward the brush 27a from the spray opening 87a of the nozzle 84a (steps S9 and S10).

On completion of elapse of the predetermined stop period, the controller 26 perform the control such that the rotational drive of the pump 78 is stopped, the electromagnetic valve 85a is made close, and the motor M is driven to rotate the roller 28 so that the brush 27a, that is, the brush wire 27b is pulled back through the suction channel 22a (steps S11 and S12). Hence the brush 27a is returned to its initial position.

The foregoing washing operation is performed repeatedly predetermined times (step S13). After this, based on the information that was already acquired from the operation panel 8, the controller 26 automatically determines whether or not the next process is required (for example, the disinfecting process after the washing process) (step S14). If this determination shows that the next process is required, the three-way switching valves 44 and 45 are switched to internal paths adaptive to the next process (step S15), before the processing returns to step S6. In the next processing, with using another type of fluid, the processing similar to the foregoing is carried out as long as there are endoscopes being washed on the tray 10 (step S16).

Thus, the brush 27a is able to fully wash the suction channel 22a by brushing the channel wall to remove the various kinds of humor, such as mucosa and blood, and filth attached thereon. The humor and filth attached to the brush 27a are also washed away by the washing fluid sprayed from the nozzle 84a.

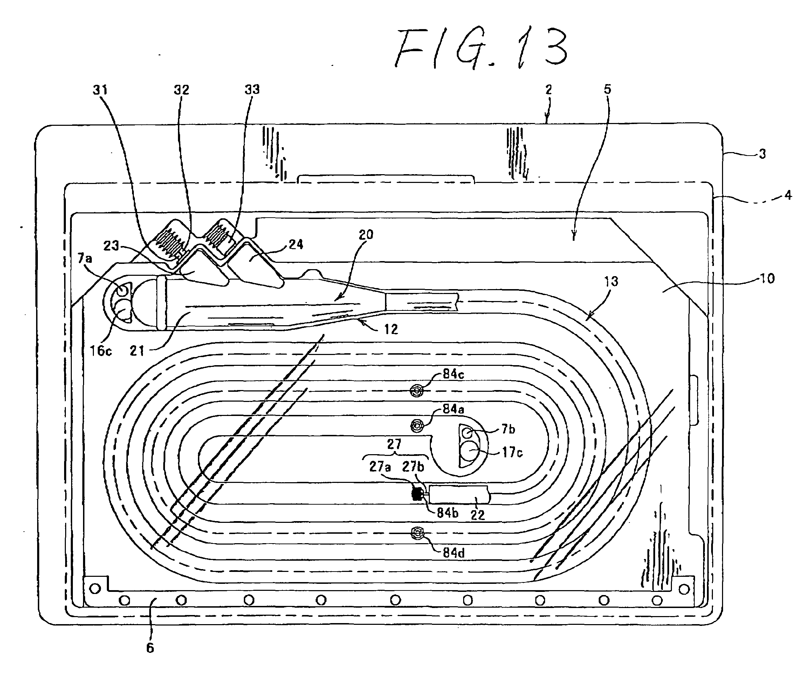

In contrast, as shown in Fig. 13 to 15, when an endoscope 20 which has a insertion tube 22 shorter than the foregoing insertion tube 22 adapted to the nozzle 84a is accommodated on the tray 10, information indicative of the length of the insertion tube 22 is interpreted from the information read from an endoscope ID medium 20a attached to the endoscope 20 is used. And the read-out length information, an appropriately located nozzle 84b (84c, 84d), which is in proximity to the distal end of the insertion tube, is specified. That is, a nozzle connector 85b (85c, 85d) corresponding to the specified nozzle is also decided. Then the controller 26 calculates a feeding amount of the brush 27a to make it advance and retreat through the suction tube 22a, and drives the motor M depending on the calculated feeding amount.

Like the case for the nozzle 84a, the feeding amount is set such that the brush 27a which advances to the fullest is located just above the nozzle 84b (84c, 84d).
And when the brush 27a is protruded from the distal opening of the suction channel 22a and located just above the nozzle 84b (84c, 84d), that is, the spray opening 87b (87c, 87d), the motor M is stopped from being driven for a predetermined period of time, so that the roller 28 which make the brush wire 27b advance or retreat is also prohibited from rotating.

During stopping the motions of the brush 27a, the pump 78 is driven and an electromagnetic valve 85b (85c, 85d) corresponding to the specified nozzle 84b (84c, 84d) is made open, without operating the remaining valves. Hence, from the spray opening 87b (87c, 87d) of the nozzle 84b (84c, 84d), the fluid for washing, disinfecting and rinsing is sprayed out toward the brush 27a.

In this way, the brush 27a that applies the brushing operations to each suction channel 22a of the shorter insertion tubes 22 can also be washed well by the fluid sprayed from the nozzle 84b (84c, 84d). As a whole, using the endoscope washing and disinfecting apparatus 2 according to the present embodiment, the endoscope 20 can be accommodated and positioned on the tray 10, and the outer surface and the endoscopic channels, in particular, to the suction channel 22a, of the endoscope 20 can be washed and/or brushing-washed. Moreover, even if various types of endoscopes 20 with insertion tubes 22 of different lengths are to be washed, the distal ends of those insertion tubes can be located just in proximity to any of the nozzles 84a to 84d. And the brush 27a moved through the suction channel 22a can be located just above any nozzle 84a (84b, 84c, 84d) for washing, when the brush 27a is made to advance to the fullest.

Accordingly, operators (users) are released from the manual kneading and washing work with the brush 27a. Thus, the operators' washing work can be relieved, without performing the conventional manual washing work which is troublesome and time-consuming. Frequently, how to wash brushes by hand (including the time for washing each brush) varies person by person, and such variations sometimes lead to the remaining of humor and filth on the brushes. However, in the apparatus 2 of the present embodiment, the brush 27a is washed in the same automatic manner, so that the remaining humor and filth can be removed.
Moreover, the operating rate of each endoscope can be raised, because many endoscopes that have been used can be washed in succession without manually washing the brush 27a.
The foregoing embodiment can be developed into some other modifications. For example, in the endoscope washing and disinfecting apparatus 2, the nozzles 84 on the tray 10 and the nozzle connectors 85 coupled with the nozzles 84 in the washing bath 5 can be disposed at any positions. In performing such a free-positional design, information about the length of the insertion tube 22 of an endoscope 20 is acquired from an endoscope ID medium 20a adhered to the endoscope. Based on the acquired information, the controller 26 specifies a nozzle 84 disposed at the nearest position the in front of the distal end of the insertion tube 22, and stops at the position above the specified nozzle 84 for washing the brush 27a.

That is, irrespective of the lengths of insertion tubes, a nozzle which is positionally best matched to an insertion tube can be designated as one being actually driven. Thus, this modification provides the similar advantages to those gained in the foregoing embodiment.

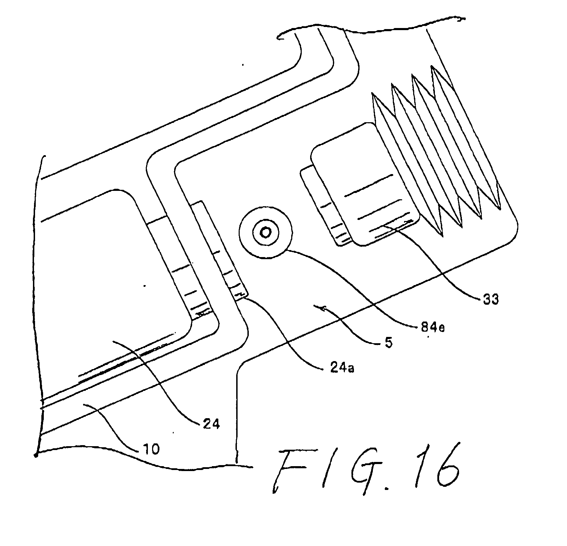

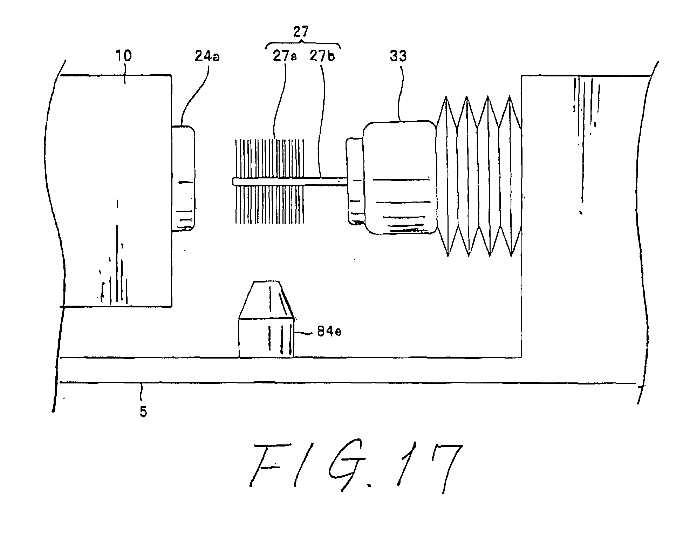

Another modification is shown In Figs. 16 and 17. As shown therein, there is a brush washing nozzle 84e is disposed at a position between the washing nozzle 33 and the suction channel port 24a of the suction channel connector 24 in the washing bath 5.

Though not shown, the nozzle 84e is directly connected to the duct 79 in which an electromagnetic valve intervenes inside the main body unit 3. This electromagnetic valve (not shown) is driven by the controller 26 so that the brush 27a is washed by fluid sprayed from the nozzle 27a at timing when the nozzle 33 is connected with or separated from the suction channel connector 24.

Accordingly, this brush washing nozzle 84e can be operated for washing the brush 27a before and/or after each of the washing, disinfecting, and rinsing processes and/or during each of the washing, disinfecting, and rinsing processes. In addition, in each process, this washing may be done, provided that the nozzle 33 is separate from the suction channel connector 24.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An apparatus for washing and disinfecting an endoscope (20) equipped with an insertion tube through which a duct (22a) is formed and a base portion (21) integrated with the insertion tube, comprising:
a bath member (3) providing a washing bath (5);
an accommodating member (10) on which the endoscope is accommodated, the accommodating member being installed in the washing bath;
a duct washing unit (16c, 17c, 27, 26, 30, 41-60, 57A, 62-70) equipped with a washing brush ( 27a) and formed to wash the duct by advancing and retreating the brush through the duct, with the endoscope accommodated on the accommodating member; and
a brush washing unit (26, 41-59, 78, 79, 84, 85) configured to wash the brush by spraying fluid toward the brush when the brush is made to advance and retreat through the duct.

2. The apparatus of claim 1, wherein
the brush washing unit is configured to spray the fluid toward the brush when the brush appears from the duct in a distal end surface of the insertion tube.

3. The apparatus of claim 2, wherein
the brush washing unit is provided a nozzle having a spray opening to spray the fluid toward the brush, a fluid supply unit supplying the fluid to the nozzle so as to make the spray opening spray the fluid to the brush, and a controller controlling timing at which the fluid supply unit is allowed to supply the fluid to the nozzle.

4. The apparatus of claim 3, wherein
the accommodating member has an accommodating surface on which the endoscope is accommodated with the insertion tube wound in a vortical form.

5. The apparatus of claim 4, wherein
the accommodating surface has a groove for guiding accommodation of the endoscope onto the accommodating member, wherein part of the groove into which the insertion tube is accommodated is shaped into the vortical form.

6. The apparatus of claim 5, wherein
the nozzle is located at a position along the groove and near a distal end of the insertion tube accommodated in the groove.

7. The apparatus of claim 6, wherein
the nozzle is disposed on the accommodating member.

8. The apparatus of claim 6, wherein
the nozzle is disposed in the washing bath so as to allow the nozzle to protrude from or open through the accommodating surface, the spray opening being formed at a top of the nozzle.

9. The apparatus of claim 6, wherein
the nozzle is composed of a plurality of nozzles located mutually differently and, respectively, located near plural distal ends of plural insertion tubes having different lengths, the plural insertion tubes being provided by a plurality of types of endoscopes.

10. The apparatus of claim 9, wherein
the plurality of nozzles is located along a linear line intersecting with orbiting groove portions forming the vertical groove.

11. The apparatus of claim 1, wherein
the brush washing unit comprises a nozzle having a spray opening through which the fluid is sprayed toward the brush, wherein
the nozzle is disposed at a position in the washing bath, the position being near an opening of the base portion of the endoscope accommodated on the accommodating member, the brush being inserted into the duct from the opening leading to the duct.

12. The apparatus of claim 1, wherein the accommodating member is detachable to and from the bath member.

13. The apparatus of claim 3, wherein
the nozzle is composed of a plurality of nozzles located mutually differently and, respectively, located near plural distal ends of plural insertion tubes having different lengths, the plural insertion tubes being provided by a plurality of types of endoscopes.

14. The apparatus of claim 13, wherein
the controller comprises means for acquiring information indicating that the endoscope is of which type, means for determining a length of the insertion tube of the endoscope based on the acquired information, and means for selecting a desired nozzle from the plural nozzles based on the length of the insertion tube.
